# EUROPEAN PATENT APPLICATION

(11) **EP 4 494 621 A1**
(43) Date of publication of application: **22.01.2025**
(21) Application number: 23826989.8
(22) Date of filing: 07.06.2023
(51) Int. Cl.: A61F 13/49, A61F 13/496, A61F 13/51, A61F 13/551

(54) **PANT-TYPE ABSORBENT ARTICLE**

(30) Priority: 20.06.2022 JP 2022099150
(71) Applicant: Unicharm Corporation, Ehime 799-0111 (JP)
(72) Inventor: SHIMAZU, Takeshi, Kanonji-shi, Kagawa 769-1602 (JP); TANAKA, Suguru, Kanonji-shi, Kagawa 769-1602 (JP); ISHII, Yudai, Kanonji-shi, Kagawa 769-1602 (JP)
(74) Representative: Dolleymores
(86) International application number: PCT/JP2023/021198
(87) International publication number: WO 2023/248802

(57) **Abstract**

Provided is a pant-type absorbent article (1) comprising a frontside torso part (FA) and a back-side torso part (BA) joined by a pair of side joint parts (40, 40). A torso member (20) has a plurality of elastic members (25) stretchable in the left-right direction. Each of the elastic members (25) is held between two bonding portions (50) adjacent to each other in the up-down direction, so that the elastic members (25) are positionally restricted in the up-down direction. In a region between the pair of side joint parts (40, 40) in the left-right direction and between the upper and lower ends of the side joint parts (40) in the up-down direction, the total area of the bonding portions (50) provided on the upper side (FA1) of a middle position (FAC) in the up-down direction of the side joint parts (40) is greater than the total area of the bonding portions (50) provided on the lower side (FA2).

## Description

### FIELD

The present invention relates to an underpants-shaped absorbent article.

### BACKGROUND

Conventionally, there are known underpants-shaped absorbent articles in which a waist member has a plurality of elastic members that have the stretchability in the lateral direction. For example, Patent Document 1 discloses an underpants-shaped diaper in which a hot-melt adhesive is applied to the outer surfaces of elastic stretch members 12C, which are sandwiched between an outer layer 12S and an inner layer 12H that constitute an exterior sheet 12 (corresponding to the waist member), and the two layers 12S and 12H are bonded together with the adhesive and the elastic stretch members 12C.

### [CITATION LIST]

### [PATENT LITERATURE]

[Patent Document 1] Japanese Patent Application Publication No. 2012-61348

### SUMMARY

### [TECHNICAL PROBLEM]

When putting on such underpants-shaped diapers, the action of holding the waist member and pulling it up is carried out. However, when using the diaper in a manner that requires the waist member to be repeatedly raised and lowered, for example, due to the use of a pad at the same time, there are cases where the durability is insufficient in the upper portion of the waist member, which is easily held by the hand, resulting in crumpling. On the other hand, in the case where the joining strength of the materials that constitute the waist member is increased in order to secure the durability for the waist member, the stiffness of the lower portion of the waist member, which is less likely to have problems such as crumpling, will also become unnecessarily high, which could cause problems for the texture.

The present invention was achieved in light of conventional problems such as that described above, an aspect of the present invention is to provide an underpants-shaped absorbent article that includes the waist member in which its upper portion in the vertical direction has a high durability, and its lower portion in the vertical direction has a good texture.

### [SOLUTION TO PROBLEM]

A main aspect of the present invention of achieving the above-described aspect is an underpants-shaped absorbent article having a vertical direction, a lateral direction, and a front-back direction that intersect with each other, the underpants-shaped absorbent article including: a liquid-absorbent absorbent main body; and a waist member, the waist member having a front waist portion provided on a front side in the front-back direction and a back waist portion provided on a back side in the front-back direction, the front waist portion and the back waist portion being joined to each other by a pair of side joining portions that are provided on two sides in the lateral direction, the waist member having a plurality of elastic members spaced apart in the vertical direction between a first sheet and a second sheet, the plurality of elastic members being capable of stretching and contracting in the lateral direction, the first sheet and the second sheet being joined by a plurality of welded portions, in at least one of the front waist portion and the back waist portion, in a region that is located between the pair of the side joining portions in the lateral direction and that is located between upper ends and lower ends of the side joining portions in the vertical direction, a total area of the welded portion located above an intermediate position, in the vertical direction, of the side joining portion being greater than a total area of the welded portion located below the intermediate position.

Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article that includes the waist member in which its upper portion in the vertical direction has a high durability, and its lower portion in the vertical direction has a good texture.

### BRIEF DESCRIPTION OF DRAWINGS

FIG. 1 is a schematic perspective view showing an example of the configuration of a diaper 1.
FIG. 2 is a schematic plan view of the diaper 1 in a stretched state as viewed from a skin side in a thickness direction.
FIG. 3 is a schematic cross-sectional view taken along line A-A of FIG. 2.
FIG. 4 is a plan view and a cross-sectional view of an absorbent main body 10.
FIG. 5 is a diagram illustrating the range in a waist member 20 within which a plurality of welded portions 50 are formed.
FIG. 6 is a diagram illustrating an example of the arrangement of the welded portions 50.
FIGS. 7A and 7B are diagrams illustrating the principle by which waist elastic members 25 are attached to the waist member 20 by the welded portions 50.
FIG. 8 is a schematic cross-sectional view of the diaper 1 in an underpants-shaped state as viewed from a one side in the lateral direction.
FIG. 9 is a plan view illustrating the arrangement of a main-body joining portion 90 by which the absorbent main body 10 is joined to the waist member 20.
FIG. 10 is a schematic cross-sectional view of a modified example of the diaper 1 as viewed from the one side in the lateral direction.

### DESCRIPTION OF EMBODIMENTS

At least the following matters will be clear with the description of this specification and the attached drawings.

### (Aspect 1)

An underpants-shaped absorbent article having a vertical direction, a lateral direction, and a front-back direction that intersect with each other, the underpants-shaped absorbent article including: a liquid-absorbent absorbent main body; and a waist member, the waist member having a front waist portion provided on a front side in the front-back direction and a back waist portion provided on a back side in the front-back direction, the front waist portion and the back waist portion being joined to each other by a pair of side joining portions that are provided on two sides in the lateral direction, the waist member having a plurality of elastic members spaced apart in the vertical direction between a first sheet and a second sheet, the plurality of elastic members being capable of stretching and contracting in the lateral direction, the first sheet and the second sheet being joined by a plurality of welded portions, in at least one of the front waist portion and the back waist portion, in a region that is located between the pair of the side joining portions in the lateral direction and that is located between upper ends and lower ends of the side joining portions in the vertical direction, a total area of the welded portion located above an intermediate position, in the vertical direction, of the side joining portion being greater than a total area of the welded portion located below the intermediate position.

According to the underpants-shaped absorbent article of aspect 1, in the part of the waist portion above the center position in the vertical direction, the total area of the welded portion is relatively larger, so the joining strength and stiffness of the waist member are more easily secured, and the durability is improved. On the other hand, in the part below the center position, the total area of the welded portion is relatively small, so the stiffness of the waist member is low and the flexibility is high, making the texture good.

### (Aspect 2)

The underpants-shaped absorbent article according to aspect 1, wherein in at least one of the front waist portion and the back waist portion, in a region that is located between the pair of the side joining portion in the lateral direction and that is located between an upper end and a lower end of the side joining portion in the vertical direction, a total number of the welded portions located above the intermediate position, in the vertical direction, of the side joining portion is greater than a total number of the welded portion located below the intermediate position.

According to the underpants-shaped absorbent article of aspect 2, in the upper portion of the waist portion, which has a relatively large number of the welded portions, the durability of the waist member becomes higher, making it less likely to cause crumpling and the like. On the other hand, in the case of the lower portion of the waist portion, which has a relatively small number of the welded portions, the flexibility of the waist member is high, and the texture can be made good.

### (Aspect 3)

The underpants-shaped absorbent article according to aspect 1 or 2, wherein in at least one of the front waist portion and the back waist portion, in a region that is located between the pair of the side joining portions in the lateral direction and that is located between upper ends and lower ends of the side joining portions in the vertical direction, a number of sheet members stacked in the thickness direction above the intermediate position, in the vertical direction, of the side joining portions is greater than a number of sheets of the sheet member stacked on the thickness direction below the intermediate position.

According to the underpants-shaped absorbent article of aspect 3, in the upper portion of the waist portion, which has a larger number of stacked sheet members, the stiffness increases, which can improve the durability. On the other hand, in lower portion of the waist portion where the number of stacked sheet members is smaller, the stiffness decreases, which can improve the flexibility.

### (Aspect 4)

The underpants-shaped absorbent article according to any one of aspects 1 to 3, wherein the underpants-shaped absorbent article has a fold-back portion in an upper end portion of the waist member, the fold-back portion being a portion in which the first sheet and the second sheet that are overlaid are folded together from above to below in the vertical direction.

According to the underpants-shaped absorbent article of aspect 4, the presence of the fold-back portion allows the number of sheet members stacked in the upper end portion of the waist member can be increased, thereby increasing the stiffness of the upper end portion and making it easier to improve the durability.

### (Aspect 5)

The underpants-shaped absorbent article according to aspect 4, wherein in the fold-back portion, at least parts of the first sheet and the second sheet are joined to each other by the welded portion.

According to the underpants-shaped absorbent article of aspect 5, in the fold-back portion, the integrity of the sheet members is increased and the presence of the welded portion increases the stiffness. Accordingly, the stiffness of the upper end portion of the waist member increases, making it easier to improve the durability.

### (Aspect 6)

The underpants-shaped absorbent article according to any one of aspects 1 to 5, wherein in at least one of the front waist portion and the back waist portion, a non-existent region where the welded portion does not exist is provided in a region having a predetermined width and extending upward from a lower end position of the side joining portions, in the vertical direction.

According to the underpants-shaped absorbent article of aspect 6, the non-existent region of the lower end portion of the waist portion does not have the welded portion, so compared to other regions, the stiffness of the waist member is lower and the flexibility is more likely to be secured. For example, when the underpants-shaped absorbent article is worn, the flexibility of the waist member is secured in portions that are likely to come into contact with the wearer's buttocks and the like, and this can make the wearer less likely to feel the discomfort.

### (Aspect 7)

The underpants-shaped absorbent article according to any one of aspects 1 to 6, wherein in at least one of the front waist portion and the back waist portion, in a region that is located between the pair of the side joining portions in the lateral direction and that is located between upper ends and lower ends of the side joining portions in the vertical direction, an average pitch, in the vertical direction, of a plurality of the elastic members that are located above the intermediate position, in the vertical direction, of the side joining portions is narrower than an average pitch, in the vertical direction, of a plurality of the elastic members that are located below the intermediate position.

According to the underpants-shaped absorbent article of aspect 7, the smaller the pitch in the vertical direction of the waist elastic member, the more likely it is that the welded portion pair are densely formed and the higher the stiffness of the waist member. Therefore, if the pitch of the elastic members in the upper portion of the waist portion is smaller than the pitch in the lower portion, the stiffness of the upper portion is likely to be higher than the that of the lower portion. As a result, in the upper portion, the durability of the waist member is higher, making crumpling less likely to occur. On the other hand, in the lower portion, the flexibility of the waist member becomes higher, and the texture can be made good.

### (Aspect 8)

The underpants-shaped absorbent article according to any one of aspects 1 to 7, wherein between two of the elastic members that are adjacent in the vertical direction, the welded portion that is not in contact with the two elastic members is located.

According to the underpants-shaped absorbent article in aspect 8, the welded portion is provided in portions that do not contribute to the attaching of the waist elastic member, the joining strength between the first and second sheets that make up the waist member further enhances, making it easier to improve the durability. (Aspect 9)

The underpants-shaped absorbent article according to any one of aspects 1 to 8, wherein the underpants-shaped absorbent article has a tape member located in a non-skin-side surface of at least either one of the front waist portion and the back waist portion, and in a region that is located between the pair of the side joining portions in the lateral direction and that is located between upper ends and lower ends of the side joining portions in the vertical direction, the tape member has a portion that overlaps a region located above the intermediate position of the side joining portion with respect to the vertical direction.

According to the underpants-shaped absorbent article of aspect 9, the stiffness of the waist member is locally increased in the overlapping portion with the tape member, thereby improving the durability. This makes it easier to prevent the waist member from becoming crumpled, even if the waist member is repeatedly raised and lowered while the underpants-shaped absorbent article is being worn. (Aspect 10)

The underpants-shaped absorbent article according to any one of aspects 1 to 9, wherein the underpants-shaped absorbent article has a main-body joining portion that joins the absorbent main body and the waist member, in the vertical direction, an intermittent portion is provided in which the main-body joining portion is not provided, and in a region that is located between the pair of the side joining portions in the lateral direction and that is located between upper ends and lower ends of the side joining portions in the vertical direction, the intermittent portion has a portion that overlaps a region located below the intermediate position, in the vertical direction, of the side joining portions, with respect to the vertical direction.

According to the underpants-shaped absorbent article of aspect 10, the intermittent portion is provided at a position that overlaps with the lower portion of the waist portion, and the main-body joining portion is not formed. This makes the flexibility of the waist member easier to be secured at the lower portion. (Aspect 11)

The underpants-shaped absorbent article according to any one of aspect 1 to 10, wherein in a region that is located between the pair of the side joining portions in the lateral direction and that is located between upper ends and lower ends of the side joining portions in the vertical direction, a total area of the welded portion located below the intermediate position, in the vertical direction, of the side joining portion is smaller in the back waist portion than in the front waist portion.

According to the underpants-shaped absorbent article in aspect 11, in the lower portion of the back waist portion, the flexibility of the waist member is higher compared to the lower portion of the front waist portion. This makes the underpants-shaped absorbent article soft against the skin of the wearer's buttocks when being worn, making the wearer less likely to feel the unpleasantness or the discomfort even if the wearer lies on their back or sits down while wearing the underpants-shaped absorbent article. (Aspect 12)

The underpants-shaped absorbent article according to any one of aspect 1 to 11, wherein in at least one of the front waist portion and the back waist portion, in a region that is located between the pair of the side joining portions in the lateral direction and that is located between upper ends and lower ends of the side joining portions in the vertical direction, below the intermediate position, in the vertical direction, of the side joining portions, a total area of a part where the welded portion is not located is greater than a total area of a part where the welded portion is located.

According to the underpants-shaped absorbent article in aspect 12, in the lower portion of the waist portion, by reducing the proportion (area ratio) of the welded portion, which causes the wearer to feel stiff when being worn, the flexibility of the entirety of the lower portion of the waist portion is more easily ensured. This makes the underpants-shaped absorbent article feel softer on the skin of the buttocks, making the wearer less likely to feel the unpleasantness or the discomfort. (Aspect 13)

The underpants-shaped absorbent article according to any one of aspects 1 to 12, in at least one of the front waist portion and the back waist portion, letting a length, in the vertical direction, of the fold-back portion be L, a stiffness of a region that extends downward from a lower end of the fold-back portion in the vertical direction by the length L is higher than a stiffness of a region that extends upward from the lower end of the side joining portion in the vertical direction by the length L.

According to the underpants-shaped absorbent article in aspect 13, by making the stiffness of the waist portion in the region that extends downward from the lower end of the fold-back portion by the length L higher than that of the region that extends upward from the lower end of the waist portion by the length L, it is possible to improve the durability in the region that is likely to be held and pulled when putting on (the region that extends downward from the lower end of the fold-back portion by the length L). On the other hand, since the stiffness is relatively low in the region that extends upward from the lower end of the waist portion by the length L, it is easier to secure the flexibility.

### Embodiment

As an example of the underpants-shaped absorbent article according to the embodiment of the present invention, an underpants-shaped diaper 1 (hereinafter, simply referred to as "diaper 1") will be described. In addition, the underpants-shaped absorbent article also includes a shorts-type sanitary napkin and an underpants-shaped absorbent pad.

### Basic structure of diaper 1

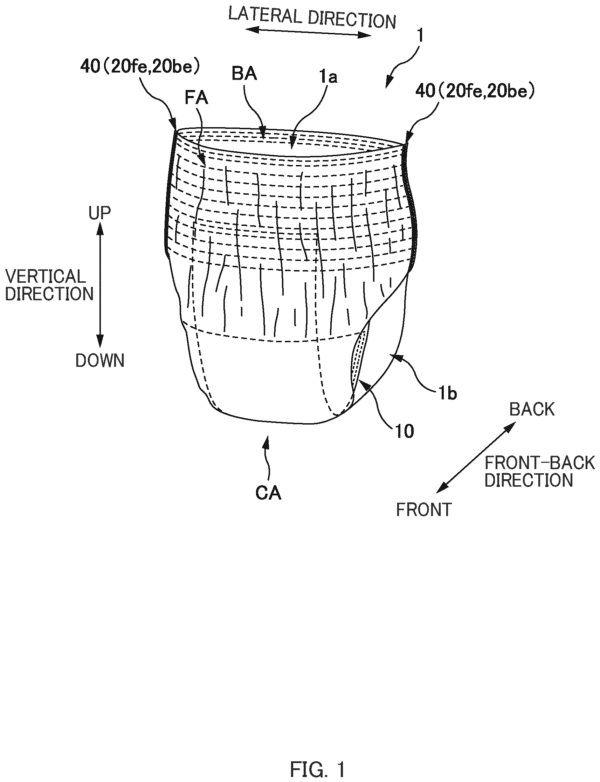

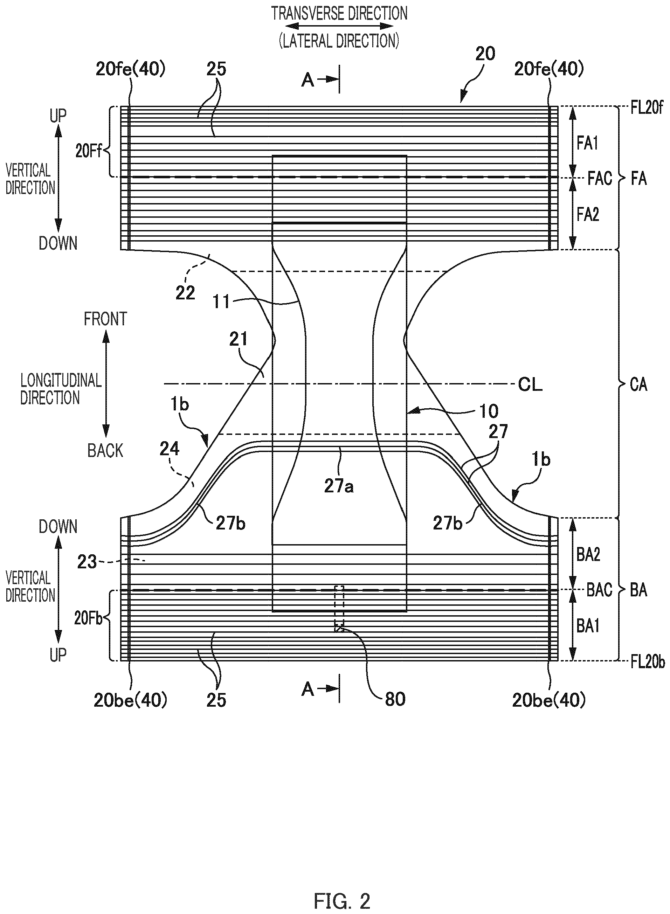

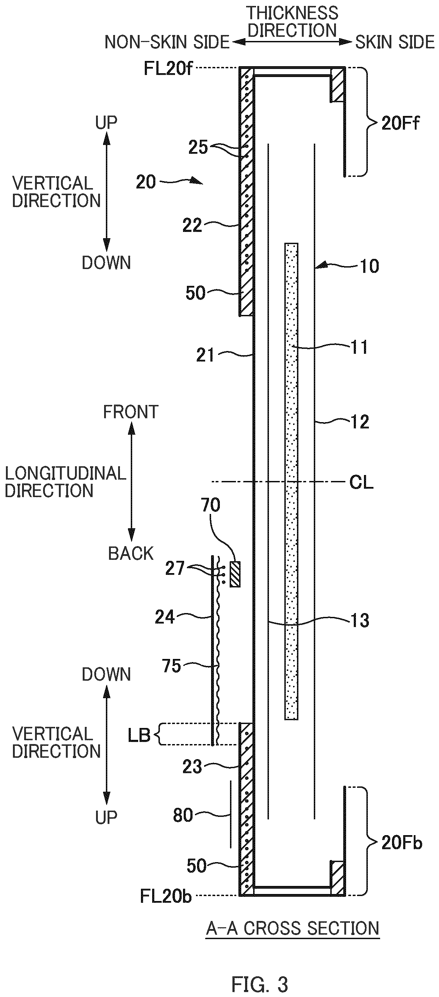

FIG. 1 is a schematic perspective view showing an example of the configuration of the diaper 1 according to the present embodiment. FIG. 2 is a schematic plan view of the diaper 1 in a stretched state as viewed from a skin side in a thickness direction. FIG. 3 is a schematic cross-sectional view taken along line A-A of FIG. 2.

In addition, the "stretched state" of the diaper 1 refers to a state where the entire diaper 1 (entire product) is stretched without wrinkles by stretching the elastic members (e.g., later-described waist elastic members 25 and leg elastic members 27) provided in the diaper 1, or more specifically, a state where the diaper 1 is stretched until the dimensions of the constituent members of the diaper 1 (e.g., a later-described absorbent main body 10 and waist member 20) match or are close to the dimensions of the members on their own. On the other hand, compared to the stretched state, the natural state refers to the state shown in FIG. 1. The "natural state" is a state where the diaper 1 has been left alone for a predetermined period. For example, the waist member 20 of the diaper 1 is pulled outward on both sides in the lateral direction to put the waist member 20 in the "stretched state", and the stretched state is maintained for 15 seconds, and then the diaper 1 is released and placed on a flat surface such as a desk. Then, the state after five minutes of being laid flat on the flat surface is defined as the natural state.

In the underpants-shaped state shown in FIG. 1, the diaper 1 has a "vertical direction", a "lateral direction", and a "front-back direction" that are orthogonal to each other. Also, as shown in FIG. 3, the diaper 1 has a "thickness direction", which is the direction in which members are overlaid on each other. When the wearer wears the diaper 1, the "upper side" in the vertical direction is the side that faces the wearer's torso, and the "lower side" in the vertical direction is the side that faces the wearer's crotch region. Also, in the put-on state, the "front side" in the front-back direction is the side that faces the wearer's abdomen, and the "back side" in the front-back direction is the side that faces the wearer's back. In addition, when the wearer wears the diaper 1, the "skin side" in the thickness direction is the side that comes into contact with the wearer's skin, and the "non-skin side" in the thickness direction is the opposite side.

In addition, in the unfolded state shown in FIG. 2, the diaper 1 has a "longitudinal direction" and a "transverse direction" that are orthogonal to each other. The "longitudinal direction" is a direction conforming to the vertical direction in the underpants-shaped state, and the "transverse direction" is a direction conforming to the lateral direction in the underpants-shaped state.

The diaper 1 of the present embodiment includes: an absorbent main body 10 that has liquid absorbency and absorbs a liquid such as urine; and the waist member 20 that is joined to the non-skin side of the absorbent main body 10 and is arranged at the wearer's waist when the diaper 1 is put on.

### Absorbent main body 10

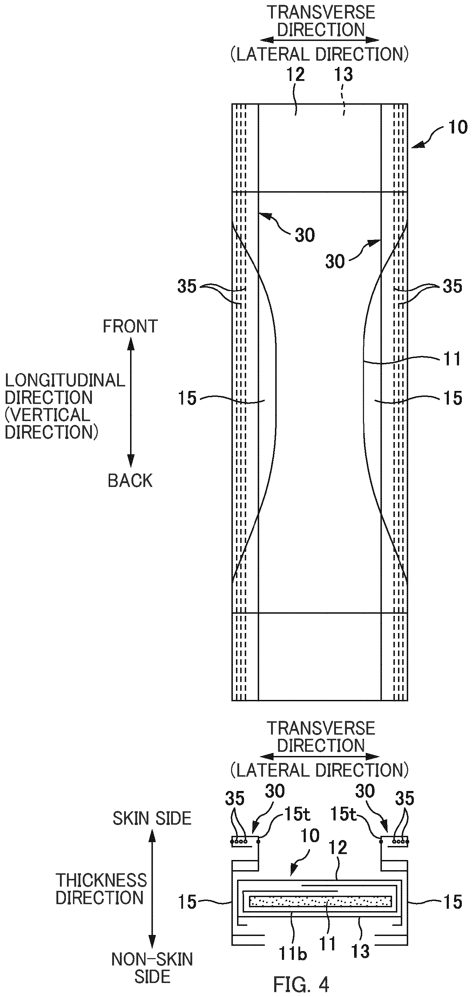

FIG. 4 is a plan view and a cross-sectional view of an absorbent main body 10. As shown in FIG. 2, the absorbent main body 10 of the present embodiment has a substantially rectangular shape in a plan view, and the longitudinal direction (i.e., the long side direction of the absorbent main body 10) conforms to the vertical direction of the diaper 1. As shown in FIGS. 3 and 4, the absorbent main body 10 includes an absorbent core 11 that extends in the longitudinal direction (vertical direction), a top sheet 12 arranged on the skin side with respect to the absorbent core 11, a back sheet 13 arranged on the non-skin side with respect to the absorbent core 11, and a pair of side sheets 15 and 15 arranged on both sides of the absorbent core 11 in the transverse direction (lateral direction).

The absorbent core 11 is a member that absorbs and retains excreted fluid such as urine. For example, the absorbent core 11 can be obtained by molding liquid-absorbent fibers (e.g., pulp) mixed with a superabsorbent polymer (SAP) into a predetermined shape. The absorbent core 11 of the present embodiment is shaped so as to have a substantially hourglass shape with its longitudinal central portion narrowing inward in the lateral direction, as shown in FIG. 4, but the shape of the absorbent core 11 is not limited to this. In addition, the outer circumferential surface of the absorbent core 11 may be covered by a liquid-permeable sheet member (core-wrapping sheet 11b) made of tissue paper or the like.

The top sheet 12 is a liquid-permeable sheet member that can come into contact with the wearer's skin during usage, and is made of hydrophilic air-through nonwoven fabric or the spunbond nonwoven fabric, for example. In the cross-sectional view of FIG. 4, the top sheet 12 is arranged so that its two lateral end portions are folded onto the non-skin side with respect to the absorbent core 11, and therefore the top sheet 12 wraps around and covers the absorbent core 11 from the skin side. In addition, another sheet or the like (not shown) may be provided between the top sheet 12 and the absorbent core 11.

The back sheet 13 is a liquid-impermeable and breathable sheet member (breathable sheet member) for preventing a liquid such as urine absorbed in the absorbent core 11 from leaking to the outside, and is made of a liquid-impermeable film such as polyethylene, polypropylene or the like.

Each of the side sheets 15 is a sheet member located on both transverse sides of the absorbent core 11, and is made of a hydrophobic nonwoven fabric sheet such as a SMS (spunbond-meltblown-spunbond) nonwoven fabric sheet, for example. The vertical (lateral) one-end side of the side sheet 15 is folded so as to be located on the non-skin side with respect to the absorbent core 11 and the back sheet 13. On the other hand, the vertical (lateral) other-end side of the side sheet 15 is folded so as to be located on the skin side with respect to the absorbent core 11 and the top sheet 12, forming a pair of leak-proof wall portions 30 and 30 on both sides of the absorbent main body 10.

The pair of leak-proof wall portions 30 are respectively provided on the two sides of the absorbent main body 10 in the transverse direction (lateral direction), and are portions corresponding to so-called barrier cuffs. When the diaper 1 is put on, the leak-proof wall portions 30 rise toward the wearer's skin on the two sides of the absorbent main body 10 in the lateral direction, thus suppressing the case where excreted fluid such as urine leaks along the wearer's skin to the outside of the absorbent main body 10 in the lateral direction. In addition, each of the leak-proof wall portions 30 may be composed of two or more sheet members (side sheet 15).

As shown in the cross-sectional view of FIG. 4, in the end portion 15t of the portion of the side sheet 15 that is folded toward the skin side in the thickness direction, the side sheet 15 is folded over multiple times so as to be overlaid in the thickness direction, and the leak-proof-wall elastic members 35 are provided between the overlaid portions of the side sheet 15 in a state of being stretched along the longitudinal direction. When the diaper 1 is put on, the end portions 15t of the leak-proof wall portions 30 contracts in the longitudinal direction (vertical direction) due to the stretchability exhibited by the leak-proof-wall elastic members 35, making the absorbent main body 10 likely to fit around the wearer's legs.

### Waist member 20

The waist member 20 is a sheet member located on the non-skin side of the absorbent main body 10, and has a skin-side sheet 21, non-skin-side sheets 22 and 23, and a cover sheet 24. Also, as shown in FIG. 2, the portion of the waist member 20 that overlaps the later-described side joining portion 40 provided on the front side in the longitudinal direction with respect to the center position CL (on the front side in the front-back direction in FIG. 1) is referred to as a "front waist portion FA". Similarly, the portion of the waist member 20 that overlaps the side joining portion 40 provided on the back side in the longitudinal direction with respect to the center position CL (on the back side in the front-back direction in FIG. 1) is referred to as a "back waist portion BA". In the longitudinal direction, the portion between the back end of the front waist portion FA and the front end of the back waist portion BA is referred to as a "crotch portion CA".

Furthermore, in the front waist portion FA, the portion that is located above the center position FAC of the front waist portion FA in the vertical direction is defined as a front waist upper portion FA1 (hereinafter referred to simply as the "front upper portion FA1"), and the portion that is located below the center position FAC is defined as a front waist lower portion FA2 (hereinafter referred to simply as the "front lower portion FA2") . Similarly, in the back waist portion BA, the portion that is located above the center position BAC of the back waist portion BA in the vertical direction is defined as a back waist upper portion BA1 (hereinafter referred to simply as the "back upper portion BA1"), and the portion that is ocated below the center position FAC is defined as a back waist lower portion BA2 (hereinafter referred to simply as the "back lower portion BA2") (see FIG. 2).

The skin-side sheet 21 is a sheet member that is arranged on the skin side of the waist member 20, and is made of spunbond nonwoven fabric, SMS nonwoven fabric, or the like, for example. As shown in FIG. 2, the skin-side sheet 21 extends continuously from the front side to the back side in the longitudinal direction, and has a shape narrowed inward in the lateral direction in the crotch portion CA. This narrowed portion becomes the leg openings 1b (described later) in the diaper 1 in the underpants-shaped state.

The non-skin-side sheet 22 is a sheet member overlaid on the non-skin side of the skin-side sheet 21, on the front side in the longitudinal direction. Similarly to the skin-side sheet 21, the non-skin-side sheet 22 is made of spunbond nonwoven fabric, SMS nonwoven fabric, or the like. The skin-side sheet 21 and the non-skin-side sheet 22 are overlaid in the thickness direction and joined to each other by a plurality of welded portions 50, 50, ..., which will be described later. In addition, between the skin-side sheet 21 and the non-skin-side sheet 22, a plurality of the waist elastic members 25, 25, ... are provided. The plurality of waist elastic members 25, 25,... are arranged side-by-side spaced apart in the longitudinal direction (vertical direction), and are attached between the skin-side sheet 21 and the non-skin-side sheet 22 in a state of being stretched in the transverse direction (lateral direction). As a result, the waist elastic members 25 exhibit the stretchability along the transverse direction (lateral direction), which then gives the stretchability to the front waist portion FA of the waist member 20.

The waist elastic members 25 can be made of, for example, an elastic resin material whose main component is thermoplastic elastic resin, which is molded into the shape of an elastic string. As the above-mentioned thermoplastic elastic resin, a thermoplastic resin that exhibits rubber elasticity at room temperature is preferable, for example, and an example thereof is thermoplastic elastomers described in JIS K 6418:2007. As the above-mentioned thermoplastic elastic resin, a material with a temperature range in which elastic deformation occurs of approximately 100°C or less is preferable. Examples of the above-mentioned thermoplastic elastic resin include olefin elastomers (e.g., VERSIFY (trademark) from The Dow Chemical Company), propylene elastomers (e.g., Vistamaxx (trademark) from Exxon Mobil Corporation), and styrene elastomers (e.g., Quintac (trademark) from Zeon Corporation).

The non-skin-side sheet 23 is a sheet member overlaid on the non-skin side of the skin-side sheet 21, on the back side in the longitudinal direction, and has a similar configuration to the non-skin-side sheet 22. That is, the non-skin-side sheet 23 is joined to the skin-side sheet 21 by the plurality of welded portions 50. In addition, between the skin-side sheet 21 and the non-skin-side sheet 23, a plurality of the waist elastic members 25, 25, ... are provided in a manner of being capable of stretching/contracting along the transverse direction (lateral direction).

The cover sheet 24 is a sheet member overlaid on the non-skin side of the skin-side sheet 21, similarly to the non-skin-side sheet 23, on the back side in the longitudinal direction. As shown in FIG. 3, in the diaper 1, the cover sheet 24 is located below the non-skin-side sheet 23 in the vertical direction (on the front side in the longitudinal direction), and a part thereof is arranged overlapping the non-skin-side sheet 23. Hereinafter, the portion where the cover sheet 24 and the non-skin-side sheet 23 overlap with respect to the vertical direction (longitudinal direction) is also referred to as a "back overlapping portion LB".

The cover sheet 24 is joined to a part of the skin-side sheet 21 and a part of the non-skin-side sheet 23 via the adhesive surface 75, which is made of an adhesive such as a hot-melt adhesive or the like. Between the cover sheet 24 and the skin-side sheet 21 in the thickness direction, there are provided the leg elastic members 27, which are made of elastic strings or the like (see FIG. 3). The leg elastic members 27 are attached to the waist member 20 through an adhesive portion 70, which is made up of an adhesive such as hot-melt adhesive or the like applied to a certain area of the non-skin-side surface of the skin-side sheet 21. In the present embodiment, the leg elastic members 27 each have a straight portion 27a in the transverse (lateral) center and curved portions 27b on the two sides in the transverse direction. In the straight portion 27a, the portion where the leg elastic member 27 overlaps the absorbent main body 10 (absorbent core 11) is arranged in a straight line along the transverse direction. In the curved portion 27b, the leg elastic member 27 is arranged in a curved manner along the contour of the leg opening 1b. Since substantially the entire area of the leg elastic member 27 is fixed by the adhesive portion 70, it is possible to maintain the shapes of the straight portion 27a and the curved portions 27b of the leg elastic member 27. The stretchability exhibited by the leg elastic members 27 makes the leg openings 1b likely to fit around the wearer's legs on the back side when the diaper 1 is put on, and also makes the portion of the waist member 20 covering the wearer's buttocks less likely to roll up.

In addition, in the upper end portion of the waist member 20 in the vertical direction, a part of the skin-side sheet 21 and parts of the non-skin-side sheet 22 and 23 are folded downward. In FIG. 3, the skin-side sheet 21 and the non-skin-side sheet 22 form a front fold-back portion 20Ff, which is a portion folded back downward in the vertical direction and toward the skin side in the thickness direction, at the front bending position FL20f located in the front upper end portion of the waist member 20. The front fold-back portion 20Ff is joined to at least portions of sheet members located adjacent in the thickness direction (the skin-side sheet 21 and the absorbent main body 10, the top sheet 12 in FIG. 3) with an adhesive such as a hot-melt adhesive.

Similarly, in FIG. 3, the skin-side sheet 21 and the non-skin-side sheet 23 form a back fold-back portion 20Fb, which is a portion folded back downward in the vertical direction (toward the front side in the longitudinal direction) and toward the skin side in the thickness direction, at the back bending position FL20b located in the back upper end portion of the waist member 20. The back fold-back portion 20Fb is joined to at least portions of sheet members adjacent in the thickness direction (the skin-side sheet 21 and the absorbent main body 10, the top sheet 12 in FIG. 3) with an adhesive such as a hot-melt adhesive.

In the diaper 1, the front fold-back portion 20Ff may be formed by folding back only either sheet member of the skin-side sheet 21 or the non-skin-side sheet 22, at the front bending position FL20f. Similarly, the back fold-back portion 20Fb may be formed by folding back only either sheet member of the skin-side sheet 21 or the non-skin-side sheet 23, at the back bending position FL20b. That is, the fold-back portions 20Ff and 20Fb each may be formed by one sheet member.

Also, a separate sheet member may be provided on the skin side with respect to the fold-back portions 20Ff and 20Fb. For example, by arranging this sheet member so that it straddles the vertical lower ends of the fold-back portions 20Ff and 20Fb in the vertical direction from the skin side (not shown in FIG. 3), it is possible to prevent the lower end edges of the fold-back portions 20Ff and 20Fb from digging into the wearer's skin when the diaper 1 is put on.

The diaper 1 is folded one time from the unfolded state shown in FIG. 2, and at this stage, the absorbent main body 10 and the waist member 20 is folded in the longitudinal direction at a bending position, which is the longitudinal center position CL (shown as the single-dotted chain line in FIG. 2). In this folded state, of the waist member 20, the front waist portion FA and the back waist portion BA are overlaid on each other in the front-back direction. The transverse side portions 20fe and 20fe of the front waist portion FA and the transverse side portions 20be and 20be of the back waist portion BA are joined together using a known joining means such as seal welding, thus forming a pair of the side joining portions 40 and 40. As a result, the two portions of the folded waist member 20 are connected on the front side and the back side to form a ring, thus forming one waist opening 1a and the pair of leg openings 1b as shown in FIG. 1, and obtaining the underpants-shaped diaper 1.

### Attaching of waist elastic members 25

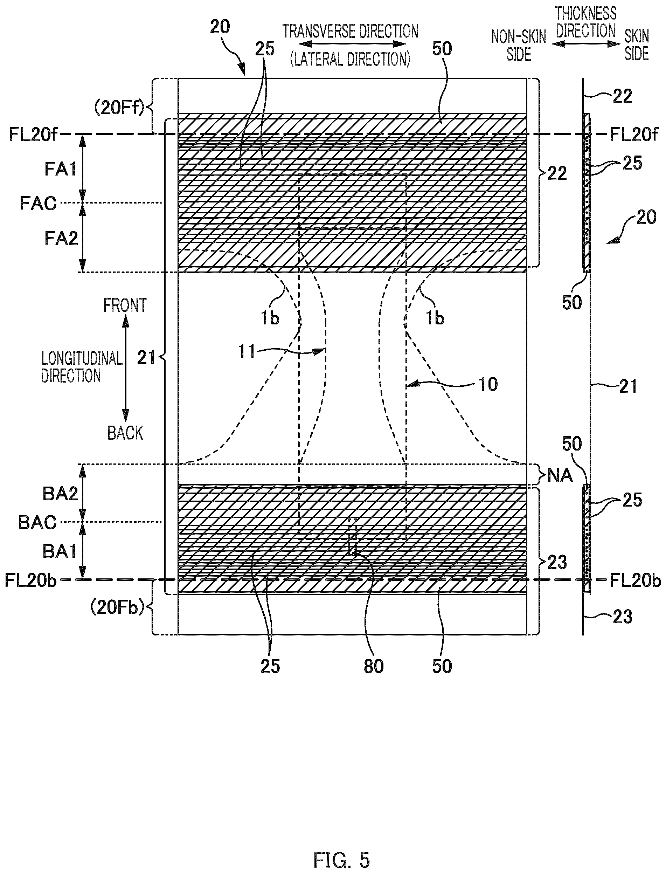

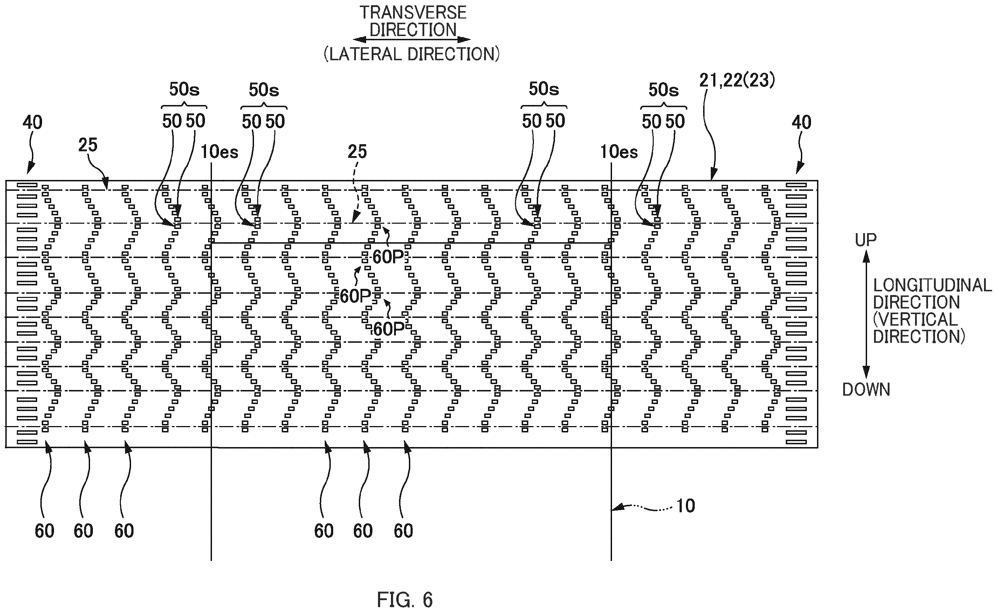

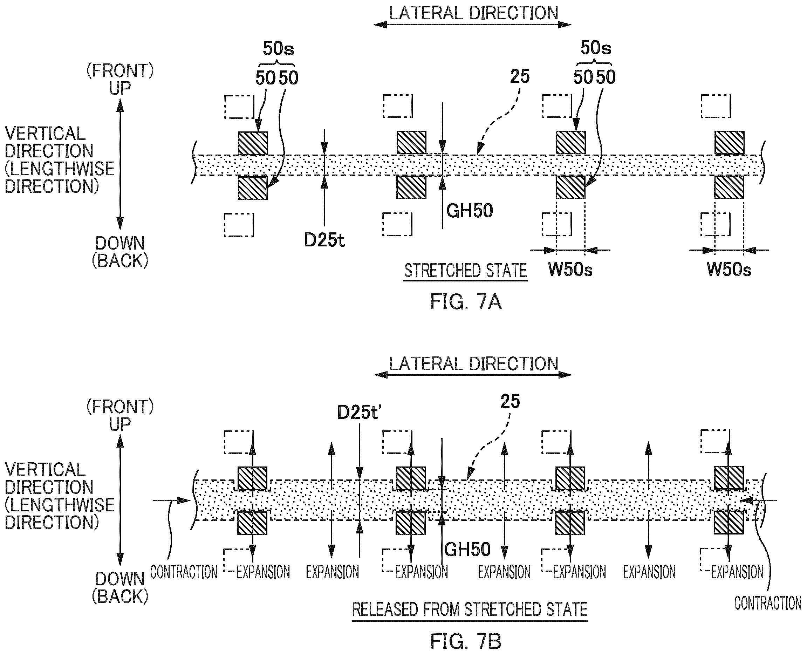

The specific method for attaching the waist elastic members 25 and the leg elastic members 27 to the waist member 20 will be explained. First, the method for attaching the waist elastic members 25 will be described. FIG. 5 is a diagram illustrating the range in the waist member 20 within which a plurality of the welded portions 50 are formed. FIG. 6 is a diagram illustrating an example of the arrangement of the welded portions 50. FIGS. 7A and 7B are diagrams illustrating the principle by which the waist elastic members 25 are attached to the waist member 20 by the welded portions 50.

In the waist member 20 of the diaper 1, the skin-side sheet 21 and the non-skin-side sheets 22 and 23 are joined by the plurality of welded portions 50, 50, ... that are formed by using a known welding means such as ultrasonic welding. In FIG. 5, the hatched portion is the region where the welded portions 50 are formed when the non-skin-side sheets 22 and 23 (corresponding to the second sheet) are joined to the non-skin side of the skin-side sheet 21 (corresponding to the first sheet) during the manufacturing process of diaper 1. At the stage of joining the skin-side sheet 21 and the non-skin-side sheets 22 and 23, the leg openings 1b have not yet been formed (the sheet member has not yet been cut). In addition, in FIG. 5, the position of the absorbent main body 10 (absorbent core 11) is indicated by a dotted line, but at the stage of forming the welded portions 50, the absorbent main body 10 has not yet been joined to the waist member 20.

As shown in FIG. 5, the skin-side sheet 21 and the non-skin-side sheets 22 and 23 are joined in the portions where they overlap in the thickness direction in the stretched state before being folded at the front bending position FL 20f and the back bending position FL 20b. That is, the welded portions 50 are formed in ranges respectively straddling the bending positions FL20f and FL20b in the front-back direction. In the range indicated by the hatched portions in FIG. 5, between the skin-side sheet 21 (first sheet) and the non-skin-side sheets 22 and 23 (second sheet) that face each other in the thickness direction, the waist elastic members 25, 25, ... which can stretch/contract along the lateral direction are attached due to the welded portions 50, side-by-side spaced apart in the vertical direction (longitudinal direction).

In the vertical direction, in a region extending upward from the lower end of the back waist portion BA and having a predetermined width (i.e. the lower end portion of the back lower portion BA2), a non-existent region NA is provided where the welded portion 50 does not exist. This non-existent region NA is a region that comes into contact with the wearer's buttocks when the diaper 1 is worn. Since the region NA does not have the welded portion 50 formed therein, the flexibility is easily secured and the texture on the buttocks side can be made softer.

In the waist member 20 of the present embodiment, as shown in FIG. 6, the plurality of welded portions 50, 50, ... are arranged side-by-side spaced apart in the vertical direction (longitudinal direction), thereby forming a welded portion row 60 extending along the vertical direction. A plurality of the welded portion rows 60 are provided spaced apart in the lateral direction (transverse direction). In FIG. 6, for the sake of simplicity, the arrangement and quantities of the waist elastic members 25 and the welded portions 50 are shown schematically. Each welded portion row 60 is arranged so as to have convex portions 60P that are convex on the one side or the other side in the lateral direction. That is, since the lateral positions of the plurality of welded portions 50 included in the welded portion row 60 are different from one another, the welded portion row 60 is formed to have projections and recesses as a whole. In the example of FIG. 6, the welded portion row 60 is formed extending from top to bottom in the vertical direction so as to meander leftward and rightward. However, the arrangement of each of the welded portions 50 is not limited to the example of FIG. 6, and the welded portion row 60 may have convex portions 60P only on the one side in the lateral direction. Also, the welded portion row 60 may be arranged in a straight line along the vertical direction without having any bumps in the lateral direction.

Of the plurality of welded portions 50, 50, ... included in the welded portion row 60, the waist elastic member 25 is sandwiched above and below between the two welded portions 50 and 50 adjacent in the vertical direction, thereby attaching the waist elastic member 25 to the waist member 20. That is, the pair of welded portions 50 and 50 arranged on both vertical sides of the waist elastic member 25 form a welded portion pair 50s, and the waist elastic member 25 is attached by the welded portion pair 50s.

As shown in FIG. 7A, a pair of the welded portions 50 and 50 that form the welded portion pair 50s are arranged with a gap GH50 therebetween in the vertical direction. The size of the gap GH50 is set to be equal to or slightly larger than the outer diameter D25t of the waist elastic member 25 (e.g., an elastic string) in a state of being stretched to a target stretch factor in the lateral direction (GH50 ≥ D25t). That is, the waist elastic member 25 in the stretched state is placed between the welded portion pair 50s in the vertical direction. With such a configuration, in the manufacturing process of the diaper 1, the waist elastic members 25 in the stretched state are placed between the skin-side sheet 21 and the non-skin-side sheet 22 (23), and then when the skin-side sheet 21 and the non-skin-side sheet 22 (23) are joined, it is possible to form the welded portions 50 without overlapping the waist elastic members 25. Here, the "stretch factor" refers to the value R (= L1/L0) that indicates the ratio of the total length L1 of the stretched waist elastic member 25 to the total length L0 in a natural no-load state.

Then, when the waist elastic member 25 is relaxed from the stretched state, as shown in FIG. 7B, the waist elastic member 25 expands in the vertical direction while contracting in the lateral direction, and the outer diameter D25t' after expansion becomes larger than the gap GH50 of the welded portion pair 50s in the vertical direction (D25t' > GH50). Therefore, the expansion of the waist elastic member 25 in the vertical direction is restricted between the welded portion pair 50s, and as a result, the waist elastic member 25 is substantially sandwiched between the welded portions 50 and 50 in the vertical direction. As a result, the welded portion pair 50s restricts the positions of the waist elastic member 25 in the vertical direction and the lateral direction. Accordingly the waist elastic member 25 is attached to the waist member 20 in a manner of being capable of stretching/contracting, and the waist member 20 is given the stretchability in the lateral direction.

In addition, in the diaper 1 in the underpants-shaped state, the waist elastic member 25 is joined by the side joining portions 40 in the two lateral end portions of the waist member 20, and therefore even if the waist member 20 is stretched in the lateral direction when the diaper 1 is put on, the waist elastic member 25 does not come off the waist member 20.

### Durability and flexibility of waist member 20

When putting on the underpants-shaped absorbent article such as the diaper 1, the wearer's legs are passed through the pair of the leg openings 1b and 1b, and the waist member 20 and the absorbent main body 10 are pulled up from the toes to the crotch. At this time, the upper end portion of the waist member 20 is often held by hand and pulled upward. Therefore, it is necessary to make sure the waist member 20 is not easily torn or damaged even if it is pulled. For example, when an absorbent pad is used together with the diaper 1, it is expected that the waist member 20 will be repeatedly raised and lowered to change the absorbent pad while using the diaper 1 for a long period of time, and therefore there is a risk that the upper end portion of the waist member 20 to easily become crumpled. Therefore, it is desirable that the waist member 20 has a sufficient durability by means such as increasing the stiffness in the upper end portion.

On the other hand, in the case where the stiffness of the waist member 20 is too high, there is a risk that the texture at the portion that comes into contact with the wearer's skin when the diaper 1 is worn deteriorate, resulting in the discomfort being imparted to the wearer. Therefore, the lower end portion in the waist member 20 is less likely to cause a problem such as crumpling and is less likely to require the durability than the upper end portion, and it is desirable to have a certain degree of the flexibility.

However, in conventional underpants-shaped diapers, a plurality of elastic members are placed between the skin-side sheet and the non-skin-side sheet that make up the waist member, and the elastic members are fixed uniformly, so that the entire waist member has a uniform stiffness. This causes problems that, in the case where the waist member as a whole has a high stiffness, the texture deteriorates, and that, in the case where the waist member as a whole has a low stiffness, the upper end portion lacks the durability and makes it likely to cause crumpling.

In contrast, in the diaper 1 of the present embodiment, as described above, the waist elastic member 25 is attached to the waist member 20 by a plurality of the welded portions 50 that are disposed in a dispersed manner. The inventor discovered that in particular with a diaper 1 having such a configuration, crumpling and the rubber coming loose would occur if the diaper is raised and lowered repeatedly, but not if it is pulled up once, and that the problem could be solved by changing the area of the welded portions 50 that hold the waist elastic member 25 above and below in the waist member 20. In other words, by appropriately adjusting the arrangement of the welded portions 50, the waist member 20 can achieve both the durability and a good texture.

Specifically, in the diaper 1, the total area of the multiple welded portions 50, 50... provided in the upper region, in the vertical direction, of the waist member 20 is made larger than the total area of the multiple welded portions 50, 50... provided in the lower region. That is, in the region of the waist member 20 that is located between the pair of side joining portions 40 and 40 in the lateral direction and that is located between the upper and lower ends of the side joining portions 40 in the vertical direction (i.e., the region corresponding to the front waist portion FA and the back waist portion BA in FIG. 2), the total area of the welded portions 50 provided in the front upper portion FA1 (back upper portion BA1) above the intermediate position FAC (BAC), in the vertical direction, of the side joining portions 40 is made larger than the total area of the welded portions 50 provided in the front lower portion FA2 (back lower portion BA2) below the intermediate position FAC (BAC).

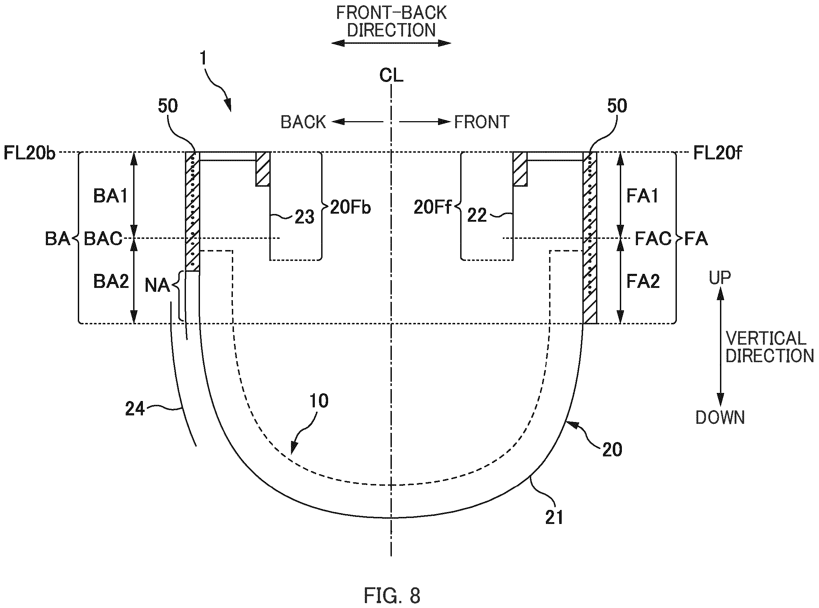

FIG. 8 is a schematic cross-sectional view of the diaper 1 in the underpants-shaped state as viewed from the one side in the lateral direction. In FIG. 8, the welded portions 50 (the welded portion row 60) is formed in the region indicated by the hatched portions of the waist member 20. Here, since the welded portions 50 are arranged in a fixed pattern as described in FIG. 6, the larger the area of the region indicated by the hatched portion in FIG. 8, the larger the total area of the welded portions 50 becomes.

Comparing the front upper portion FA1 and the front lower portion FA2 of the front waist portion FA in FIG. 8, the total area of the welded portions 50 is larger in the front upper portion FA1 by the amount of the welded portions 50 (hatched portion) provided in the front fold-back portion 20Ff. This is, as explained in FIG. 5, the welded portions 50 are formed across the front bending position FL 20f from front to back, so the welded portions 50 are also provided in part of the front fold-back portion 20Ff (the part included in the front upper portion FA1).

Therefore, in the front waist portion FA of the diaper 1, the total area of the welded portions 50 provided in the front upper portion FA1 becomes relatively larger, making it easier to ensure the joining strength and stiffness of the skin-side sheet 21 (first sheet) and the non-skin-side sheet 22 (second sheet), and improving the durability. This makes it possible to prevent crumpling and the like from occurring even if the upper end portion of the waist member 20 is repeatedly pulled up and down when being worn. On the other hand, since the total area of the welded portions 50 provided in the front lower portion FA2 is relatively small, the stiffness of the waist member 20 is lower and the flexibility is higher in the front lower portion FA2 compared to the front upper portion FA1, and the texture can be made good.

Incidentally, the welded portion 50 does not necessarily have to be arranged in a fixed pattern, and the area and shape of the welded portion 50 provided in the region indicated by the hatched portion in FIG. 8 may differ depending on the location where it is arranged. For example, by increasing the area of each of the welded portions 50 provided in the front upper portion FA1, stiffness can be more easily ensured in the front upper portion FA1, and by decreasing the area of each of the welded portions 50 provided in the front lower portion FA2, the flexibility can be made higher in the front lower portion FA2.

Next, in the case of the back waist portion BA in FIG. 8, comparing the back upper portion BA1 and the back lower portion BA2, the total area of the welded portions 50 is larger in the back upper portion BA1 than in the back lower portion BA2. This is because, like the front waist portion FA, the area of the back waist portion BA is larger by the amount of the welded portions 50 provided in the back fold-back portion 20Fb. Furthermore, the non-existent region NA in which the welded portion 50 is not provided is formed in the lower end portion of the back lower portion BA2, and therefore the total area of the welded portions 50 provided in the back lower portion BA2 is smaller. Therefore, in the back waist portion BA, the total area of the welded portions 50 provided in the back upper portion BA1 is likely to be larger than the total area of the welded portions 50 provided in the back lower portion BA2. Therefore, even in the back waist portion BA, stiffness is easily ensured in the back upper portion BA1, improving the durability. This makes it possible to prevent crumpling and the like from occurring even if the upper end portion of the waist member 20 is repeatedly pulled up and down when being worn. On the other hand, in the back lower portion BA2, the stiffness is low and the flexibility is high, so that the texture can be made good.

In the diaper 1, each of the multiple welded portions 50, 50... has the same shape and area, and the larger the area of the region indicated by the hatched portions in FIG. 5, the greater the number of the welded portions 50 provided in that region. Therefore, in the front waist portion FA (the back waist portion BA) of the diaper 1, the total number of the welded portions 50 provided in the front upper portion FA1 (back upper portion BA1) is greater than the total number of the welded portions 50 provided in the front lower portion FA2 (back lower portion). As a result, in the front upper portion FA1 (back upper portion BA1) where the number of the welded portion 50 is relatively large, the durability of the waist member 20 becomes higher, making it less likely to cause crumpling and the like. On the other hand, in the front lower portion FA2 (back lower portion BA2) where the number of the welded portions 50 is relatively small, the flexibility of the waist member 20 is high, and the texture can be made good.

In addition, since the upper end portion of the waist member 20 has the front and back fold-back portions 20Ff and 20Fb, the number of sheet members stacked in the thickness direction in the upper end portion of the waist member 20 is greater than the number of sheet members stacked in the thickness direction in the lower end portion. For example, in FIG. 8, three to four sheet members are stacked in the front upper portion FA1 of the front waist portion FA, whereas two to three sheet members are stacked in the front lower portion FA2. Therefore, in the upper end portion of the waist member 20, where the number of stacked sheet member is greater, the stiffness increases, which can improve the durability. On the other hand, in the lower end portion of the waist member 20, where the number of stacked sheet members is smaller, the stiffness decreases, which can improve the flexibility.

In addition, the fold-back portions 20Ff and 20Fb of the diaper 1 are formed by folding back from above to below at the bending positions FL20f and FL20b located at the upper end of the waist member 20 while the skin-side sheet 21 and the non-skin-side sheet 22 (23) are stacked together. By providing such fold-back portions 20Ff and 20Fb, the number of sheet members stacked in the upper end portion of the waist member 20 can be increased, thereby increasing the stiffness of the upper end portion and making it easier to improve the durability.

Furthermore, in the fold-back portions 20Ff (20Fb), the skin-side sheet 21 and the non-skin-side sheet 22 (23) stacked in the thickness direction are joined to each other by the welded portions 50. Therefore, in the fold-back portions 20Ff and 20Fb, the integrity of the sheet members is increased and the presence of the welded portions 50 increases the stiffness. Accordingly, the stiffness of the upper end portion of the waist member 20 further increases, making it easier to improve the durability.

In addition, in the back waist portion BA of the waist member 20, the non-existent region NA where the welded portion 50 does not exist is provided in a region having a predetermined width and extending upward from the lower end position of the back lower portion BA2 in the vertical direction (i.e., the lower end positions of the side joining portions 40). In the non-existent region NA, since the welded portion 50 is not provided, the stiffness of the waist member 20 is lower compared to other regions of the back waist portion BA, making it easier to secure the flexibility. In particular, the lower end portion of the back waist portion BA is a portion that is likely to come into contact with the wearer's buttocks when diaper 1 is worn, and therefore by securing the flexibility of the waist member 20 in the lower end portion of the back lower portion BA2, the wearer can be less likely to feel the discomfort.

Further, the waist member 20 of the diaper 1 has a plurality of the waist elastic members 25, 25... attached thereto, and it is desirable that the average pitch, in the vertical direction, of the waist elastic member 25 is narrower in the upper portion than in the lower portion of the waist member 20. For example, in the back waist portion BA in FIG. 2, the average of the center-to-center distances between two waist elastic members 25 and 25 located adjacent in the vertical direction (i.e., the pitch in the vertical direction) among the multiple waist elastic members 25, 25... arranged in the back upper portion BA1 is smaller than the average of the center-to-center distances between two waist elastic members 25 and 25 located adjacent in the vertical direction (the pitch) among the multiple waist elastic members 25, 25... arranged in the back lower portion BA2.

In the present embodiment, as described with reference to FIGS. 6 and 7, each of the waist elastic members 25 is attached to the waist member 20 by being sandwiched above and below by the welded portion pairs 50s. Therefore, the smaller the pitch in the vertical direction of the waist elastic members 25, the more likely it is that the welded portion pair 50s are densely formed, and the higher the stiffness of the waist member 20. Therefore, if the pitch of the waist elastic members 25 in the back upper portion BA1 is smaller than the pitch of the waist elastic members 25 in the back lower portion BA2, the stiffness of the back upper portion BA1 is likely to be higher than that of the back lower portion BA2. As a result, in the upper portion (back upper portion BA1), the durability of the waist member 20 is higher, making crumpling less likely to occur. On the other hand, in the lower portion (back lower portion BA2), the flexibility of the waist member 20 becomes higher, and the texture can be made good.

In addition, between two adjacent waist elastic members 25 and 25 in the vertical direction, there are one or more welded portions 50 that are not in contact with these waist elastic members 25 (see FIG. 6). In other words, there is provided the welded portion 50 that does not have the function of attaching the waist elastic member 25 by sandwiching it from above and below and that is only for joining the skin-side sheet 21 (first sheet) and the non-skin-side sheet 22 (23) (second sheets). In this way, by providing the welded portion 50 in portions that do not contribute to the attaching of the waist elastic members 25, the joining strength between the first sheet and the second sheet that make up the waist member 20 is further enhanced, making it easier to improve the durability.

In addition, in the diaper 1, when comparing the front and back of the waist member 20, the total area of the welded portions 50 provided in the back lower portion BA2 of the back waist portion BA is smaller than the total area of the welded portions 50 provided in the front lower portion FA2 of the front waist portion FA (see FIG. 8). As described above, the back lower portion BA2 of the back waist portion BA has the non-existent region NA where the welded portion 50 is not provided, so the area of the welded portions 50 is likely to be smaller compared to the front lower portion FA2. Thus, in the back lower portion BA2, the flexibility of the waist member 20 is higher compared to the front lower portion FA2. This makes the diaper 1 soft against the skin of the wearer's buttocks when being worn, making the wearer less likely to feel the unpleasantness or the discomfort even if the wearer lies on their back or sits down while wearing the diaper 1.

Furthermore, in the back lower portion BA2 of the back waist portion BA, the total area of the part where the welded portion 50 is not provided is greater than the total area of the part where the welded portion 50 is provided. In other words, the ratio of the area occupied by the welded portions 50 to the entire area of the back lower portion BA2 (area ratio) is less than 50%. In the back lower portion BA2, by reducing (to less than 50%) the area proportion of the welded portions 50, which causes the wearer to feel stiff when being worn, the flexibility of the entire back lower portion BA2 is more easily ensured. This makes the diaper 1 feel softer on the skin of the buttocks, making the wearer less likely to feel the unpleasantness or the discomfort.

### Other structures affecting durability and flexibility of waist member 20

### Post-handling tape 80

In the waist member 20 of the diaper 1, a post-handling tape 80 may be provided at a predetermined position. The post-handling tape 80 is a substantially rectangular tape-like member (tape member) elongated in the vertical direction (longitudinal direction), and is made of a resin material such as polyethylene or polypropylene, for example. In the diaper 1, as shown in FIGS. 2 and 3, the post-handling tape 80 is provided on the non-skin-side surface of the back waist portion BA of the waist member 20 (the non-skin-side surface of the non-skin-side sheet 23). The post-handling tape 80 is fixed to the waist member 20 on the one side in the vertical direction and has a sticky portion on the other side in the vertical direction. An adhesive is applied to the sticky portion, and before the diaper 1 is used, a portion of the post-handling tape 80 is in a state of being folded with the sticky portion on the inside, thus preventing exposure of the sticky portion to the outside and protecting a sticky surface.

When discarding of the diaper 1 after use, the diaper 1 is rolled up in the vertical direction such that the absorbent main body 10 faces inward, and the folded post-handling tape 80 is pulled out to expose the sticky portion, and the sticky portion side is wrapped around the diaper 1. Accordingly, the diaper 1 can be held in the rolled state, and the diaper 1 can be discarded without allowing the leakage of excrement and the like adhering to the inside (absorbent main body 10) of the diaper 1.

In the diaper 1, the back waist portion BA is provided with the post-handling tape 80 (tape member) mainly at a position overlapping with the back upper portion BA1. That is, there is a portion where the back upper portion BA1 and the post-handling tape 80 overlap with respect to the vertical direction. In the region overlapping with the post-handling tape 80, the stiffness of the back upper portion BA1 is locally increased, thereby enabling the durability to be increased. This makes it possible to easily prevent the waist member 20 from becoming crumpled even if the waist member 20 is repeatedly raised and lowered while the diaper 1 is being worn.

### Main-body joining portion 90

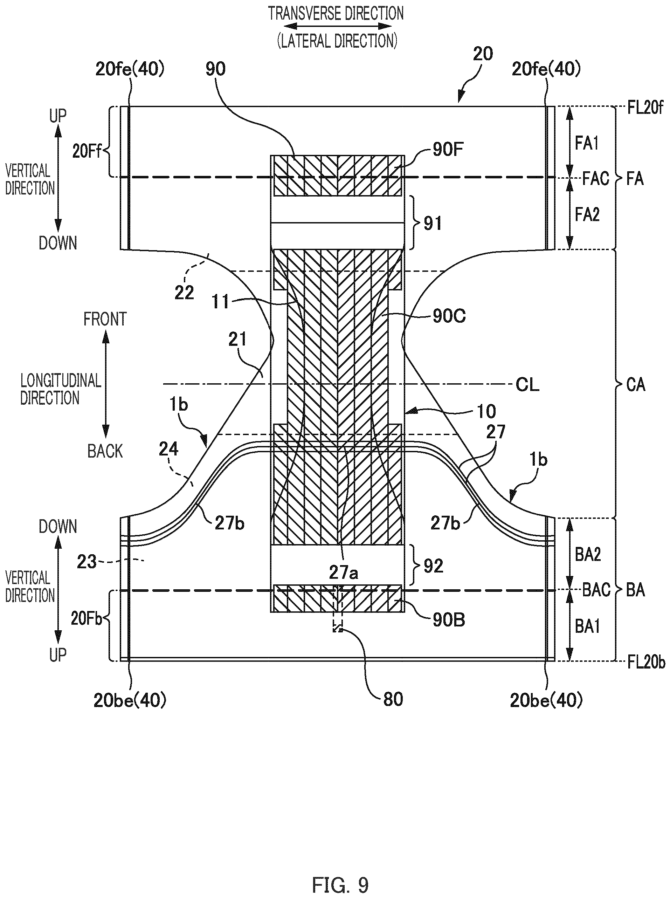

FIG. 9 is a plan view illustrating the arrangement of the main-body joining portion 90 by which the absorbent main body 10 is joined to the waist member 20. When manufacturing the diaper 1, the absorbent main body 10 is joined to the skin-side surface, in the thickness direction, of the waist member 20 at a predetermined timing in the manufacturing process. At this time, of the non-skin-side surface of the absorbent main body 10 (or the skin-side surface of the waist member 20), an adhesive such as a hot-melt adhesive is applied to the regions indicated by the hatched portions in FIG. 9, thereby forming the main-body joining portion 90. Accordingly, the waist member 20 and the absorbent main body 10 are joined via the main-body joining portion 90.

In FIG. 9, the main-body joining portion 90 has a front main-body joining portion 90F formed on the front side in the longitudinal direction, a back main-body joining portion 90B formed on the back side, and a crotch main-body joining portion 90C formed over a wide area of the crotch portion CA. In the longitudinal direction (vertical direction), between the front main-body joining portion 90F and the crotch main-body joining portion 90C, the intermittent portion 91 is provided which is a portion where the main-body joining portion 90 is not formed. Similarly, in the longitudinal direction (the vertical direction), between the back main-body joining portion 90B and the crotch main-body joining portion 90C, the intermittent portion 92 is provided which is a portion where the main-body joining portion 90 is not formed.

The intermittent portions 91 and 92 are provided at positions overlapping the front lower portion FA2 and the back lower portion BA2, respectively, with respect to the longitudinal direction (the vertical direction) (see FIG. 9). In the portion where the main-body joining portion 90 is provided, the waist member 20 and the absorbent main body 10 are joined in the thickness direction, and therefore the stiffness of the waist member 20 is likely to be high due to the stiffness of the absorbent main body 10. In the case where the main-body joining portion 90 is provided continuously from the front end to the back end of the absorbent main body 10, the stiffness of the waist member 20 is high in the front lower portion FA2 and the back lower portion BA2, making it difficult to secure the flexibility. In contrast to this, in the present embodiment, the intermittent portion 91 and the intermittent portion 92 are provided at positions overlapping with the front lower portion FA2 and the back lower portion BA2, respectively, and the main-body joining portion 90 is not formed. As a result, in the diaper 1 of the present embodiment, the flexibility in the front lower portion FA2 and the back lower portion BA2 is easily ensured.

### Modified example

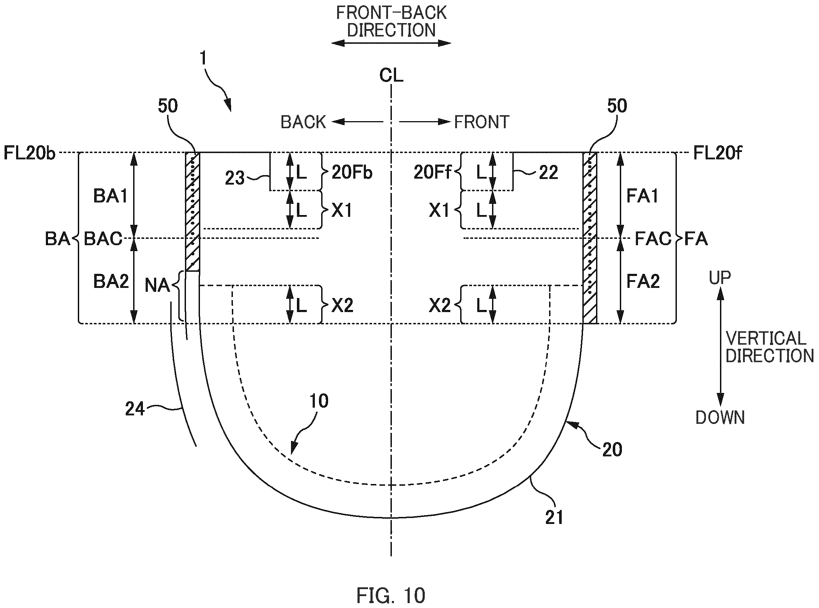

In the above-described embodiment, the upper end portion of the waist member 20 has the fold-back portions 20Ff and 20Fb, and these fold-back portions 20Ff and 20Fb respectively overlap substantially the entireties of the front upper portion FA1 and the back upper portion BA1, thereby enhancing the durability of the waist member 20 in the front upper portion FA1 and back upper portion BA1 (see FIG. 8). On the other hand, the fold-back portions 20Ff and 20Fb do not necessarily have to overlap the entire front upper portion FA1 and the entire back upper portion BA1. FIG. 10 is a schematic cross-sectional view of a modified example of the diaper 1 as viewed from the one side in the lateral direction, and corresponds to FIG. 8.

In the modified example, only one sheet of the non-skin-side sheet 22 (or the skin-side sheet 21) of the waist member 20 is folded at the front bending position FL20f to form the front fold-back portion 20Ff. And the front fold-back portion 20Ff overlaps with part of the front upper portion FA1 in the vertical direction. Similarly, only one sheet of the non-skin-side sheet 23 (or the skin-side sheet 21) is folded at the back bending position FL 20b to form the back fold-back portion 20Fb. And the back fold-back portion 20Fb overlaps with part of the back upper portion BA1 in the vertical direction. That is, compared to the case shown in FIG. 8, the fold-back portions 20Ff and 20Fb are formed shorter in the vertical direction.

In FIG. 10, letting the length, in the vertical direction, of the fold-back portion 20Ff (20Fb) be L, the region that extends downward from the lower end of the fold-back portion 20Ff (20Fb) in the vertical direction by the length L is defined as the intermediate region X1, and the region that extends upward from the lower end of the front waist portion FA (back waist portion BA) (i.e., the lower end of the side joining portion 40) in the vertical direction by the length L is defined as the lower-end region X2. In this case, it is preferable that the welded portions 50 are formed so that the stiffness of the intermediate region X1 is higher than the stiffness of the lower-end region X2. In FIG. 10, at least in the back waist portion BA, there is an overlap between the non-existent region NA, where the welded portion 50 is not provided, and the lower-end region X2. Therefore, the stiffness of the lower-end region X2 is lower than the stiffness in the intermediate region X1.

In the modified example of the diaper 1 shown in FIG. 10, the area of the fold-back portion 20Ff (20Fb) of the waist member 20 are small, and therefore the intermediate region X1 is easily held and pulled when putting on the diaper. Therefore, by making the stiffness of the intermediate region X1 higher than that of the lower-end region X2, it is possible to improve the durability of the waist member 20 in the intermediate region X1. On the other hand, by making the stiffness of the lower-end region X2 lower than that of the intermediate region X1, it becomes easier for the lower-end region X2 to secure the flexibility of the waist member 20.

### Others

The above embodiment of the present disclosure is simply to facilitate understanding of the present disclosure and is not in any way to be construed as limiting the present disclosure. The present disclosure may variously be changed or altered without departing from its gist and encompass equivalents thereof.

In the above embodiment, as an example of the underpants-shaped absorbent article, there is described the diaper 1 in which the skin-side sheet 21 of the waist member 20 is configured in an integrated manner continuously from the one side (front side) to the other side (back side) in the longitudinal direction (see FIG. 3 and the like) . But the skin-side sheet 21 may be configured as separate members on the front and back sides. For example, a so-called three-piece type underpants-shaped absorbent article may be acceptable which consists of three parts: a front exterior member (front waist portion), a back exterior member (back waist portion), and an absorbent main body.

### REFERENCE SIGNS LIST

1 diaper (underpants-shaped absorbent article),
1a waist opening, 1b leg opening,
10 absorbent main body,
11 absorbent core, 11b core-wrapping sheet,
12 top sheet, 13 back sheet, 15 side sheet, 15t end portion,
20 waist member,
20Ff front fold-back portion, 20Fb back fold-back portion,
21 skin-side sheet, 22 non-skin-side sheet, 23 non-skin-side sheet, 24 cover sheet, 25 waist elastic member,
27 leg elastic member, 27a straight portion, 27b curved portion, 30 leak-proof wall portion,
35 leak-proof-wall elastic member,
40 side joining portion,
50s welded portion, 50s welded portion pair,
60 welded portion row,
70 adhesive portion, 75 adhesive surface,
80 post-handling tape,
90 main-body joining portion
90F front main-body joining portion, back main-body joining portion, 90C crotch main-body joining portion,
91 intermittent portion, 92 intermittent portion,
E1 first elastic member, E2 second elastic member,
CL center position,
FA front waist portion, FA1 front upper portion, FA2 front lower portion, FAC intermediate position,
BA back waist portion, BA1 back upper portion, BA2 back lower portion, BAC intermediate position,
CA crotch portion,
FL20f front bending position, FL20b back bending position,
NA non-existent region.

## Claims

1. An underpants-shaped absorbent article having a vertical direction, a lateral direction, and a front-back direction that intersect with each other,
the underpants-shaped absorbent article comprising:
a liquid-absorbent absorbent main body; and
a waist member,
the waist member having
a front waist portion provided on a front side in the front-back direction and
a back waist portion provided on a back side in the front-back direction,
the front waist portion and the back waist portion being joined to each other by a pair of side joining portions that are provided on two sides in the lateral direction,
the waist member having a plurality of elastic members spaced apart in the vertical direction between a first sheet and a second sheet,
the plurality of elastic members being capable of stretching and contracting in the lateral direction,
the first sheet and the second sheet being joined by a plurality of welded portions,
each of the elastic members contracted in the lateral direction being sandwiched between two of the welded portions adjacent to each other in the vertical direction, thereby restricting a position, in the vertical direction, of the elastic member relative to the first sheet and the second sheet
in at least one of the front waist portion and the back waist portion,
in a region that is located between the pair of the side joining portions in the lateral direction and that is located between upper ends and lower ends of the side joining portions in the vertical direction,
a total area of the welded portion located above an intermediate position, in the vertical direction, of the side joining portion
being greater than
a total area of the welded portion located below the intermediate position.

2. The underpants-shaped absorbent article according to claim 1, wherein
in at least one of the front waist portion and the back waist portion,
in a region that is located between the pair of the side joining portions in the lateral direction and that is located between upper ends and lower ends of the side joining portions in the vertical direction,
a total number of the welded portions located above the intermediate position, in the vertical direction, of the side joining portion
is greater than
a total number of the welded portion located below the intermediate position.

3. The underpants-shaped absorbent article according to claim 1 or 2, wherein
in at least one of the front waist portion and the back waist portion,
in a region that is located between the pair of the side joining portions in the lateral direction and that is located between upper ends and lower ends of the side joining portions in the vertical direction,
a number of sheet members stacked in the thickness direction above the intermediate position, in the vertical direction, of the side joining portions
is greater than
a number of sheets of the sheet member stacked on the thickness direction below the intermediate position.

4. The underpants-shaped absorbent article according to claim 3, wherein
the underpants-shaped absorbent article has a fold-back portion in an upper end portion of the waist member,
the fold-back portion being a portion in which the first sheet and the second sheet that are overlaid are folded together from above to below in the vertical direction.

5. The underpants-shaped absorbent article according to claim 4, wherein
in the fold-back portion, at least parts of the first sheet and the second sheet are joined to each other by the welded portion.

6. The underpants-shaped absorbent article according to claim 3, wherein
in at least one of the front waist portion and the back waist portion,
a non-existent region where the welded portion does not exist is provided in a region having a predetermined width and extending upward from a lower end position of the side joining portions, in the vertical direction.

7. The underpants-shaped absorbent article according to claim 3, wherein
in at least one of the front waist portion and the back waist portion,
in a region that is located between the pair of the side joining portions in the lateral direction and that is located between upper ends and lower ends of the side joining portions in the vertical direction,
an average pitch, in the vertical direction, of a plurality of the elastic members that are located above the intermediate position, in the vertical direction, of the side joining portions
is narrower than
an average pitch, in the vertical direction, of a plurality of the elastic members that are located below the intermediate position.

8. The underpants-shaped absorbent article according to claim 3, wherein
between two of the elastic members that are adjacent in the vertical direction, the welded portion that is not in contact with the two elastic members is located.

9. The underpants-shaped absorbent article according to claim 3, wherein
the underpants-shaped absorbent article has a tape member located in a non-skin-side surface of at least either one of the front waist portion and the back waist portion, and
in a region that is located between the pair of the side joining portions in the lateral direction and that is located between upper ends and lower ends of the side joining portions in the vertical direction,
the tape member has a portion that overlaps a region located above the intermediate position of the side joining portions with respect to the vertical direction.

10. The underpants-shaped absorbent article according to claim 3, wherein
the underpants-shaped absorbent article has a main-body joining portion that joins the absorbent main body and the waist member,
in the vertical direction, an intermittent portion is provided in which the main-body joining portion is not provided, and
in a region that is located between the pair of the side joining portions in the lateral direction and that is located between upper ends and lower ends of the side joining portions in the vertical direction,
the intermittent portion has a portion that overlaps a region located below the intermediate position, in the vertical direction, of the side joining portions, with respect to the vertical direction.

11. The underpants-shaped absorbent article according to claim 3, wherein
in a region that is located between the pair of the side joining portions in the lateral direction and that is located between upper ends and lower ends of the side joining portions in the vertical direction,
a total area of the welded portion located below the intermediate position, in the vertical direction, of the side joining portion is smaller in the back waist portion than in the front waist portion.

12. The underpants-shaped absorbent article according to claim 3, wherein
in at least one of the front waist portion and the back waist portion,
in a region that is located between the pair of the side joining portions in the lateral direction and that is located between upper ends and lower ends of the side joining portions in the vertical direction,
below the intermediate position, in the vertical direction, of the side joining portions, a total area of a part where the welded portion is not located is greater than a total area of a part where the welded portion is located.

13. The underpants-shaped absorbent article according to claim 4, wherein
in at least one of the front waist portion and the back waist portion,
letting a length, in the vertical direction, of the fold-back portion be L,
a stiffness of a region that extends downward from a lower end of the fold-back portion in the vertical direction by the length L
is higher than
a stiffness of a region that extends upward from the lower end of the side joining portion in the vertical direction by the length L.
